# EUROPEAN PATENT APPLICATION

(11) **EP 4 620 963 A1**
(43) Date of publication of application: **24.09.2025**
(21) Application number: 23891888.2
(22) Date of filing: 03.11.2023
(51) Int. Cl.: C07K 5/11, A61P 17/00, A61K 8/64, A61Q 19/02, A61K 38/00

(54) **PEPTIDE HAVING SKIN-WHITENING ACTIVITY AND USES THEREOF**

(30) Priority: 18.11.2022 KR 20220155229
(71) Applicant: Caregen Co., Ltd., Anyang-si, Gyeonggi-do 14119 (KR)
(72) Inventor: CHUNG, Yong Ji, Anyang-si, Gyeonggi-do 14119 (KR); KIM, Eun Mi, Anyang-si, Gyeonggi-do 14119 (KR); LEE, Eung Ji, Anyang-si, Gyeonggi-do 14119 (KR); JEONG, Min Kyeong, Anyang-si, Gyeonggi-do 14119 (KR); JUNG, Da Won, Anyang-si, Gyeonggi-do 14119 (KR)
(74) Representative: Heinonen & Co
(86) International application number: PCT/KR2023/017503
(87) International publication number: WO 2024/106821

(57) **Abstract**

The peptide of the present invention has skin whitening activity due to inhibiting the incorporation of melanosomes in keratinocytes and promoting the degradation of melanosomes. The peptide of the present invention can be used as an active ingredient in drugs for the treatment or prevention of hyperpigmentation diseases caused by excessive deposition of melanosomes, or as an effective ingredient in cosmetics for skin whitening.

## Description

### Technical Field

### Cross-reference to Related Applications

This application claims the benefit of Korean Patent Application No. 10-2022-0155229, filed on November 18, 2022, in the Korean Intellectual Property Office, the disclosure of which is incorporated herein in its entirety by reference.

### TECHNICAL FIELD

The present invention relates to a peptide having activity of skin whitening and uses thereof.

### BACKGROUND ART

Skin melanin is present in the skin, hair follicles, eyes, etc. and performs an important function of protecting the skin from ultraviolet rays. However, excessive melanin formation causes hyperpigmentation diseases such as melisma, freckles, age spots, etc., thereby causing mental stress and a decrease in quality of life. Melanin is mainly synthesized in melanocytes located between the basal layers of the epidermis or below the basal layer and in hair follicles. It is synthesized in melanosomes, which are organelles within melanocytes. These melanosomes move through dendrites to adjacent keratinocytes, and skin color appears as the keratinocytes emerge into the outer skin. Although the mechanism of how melanosomes move from melanocytes to keratinocytes has not been elucidated, it is known that melanosomes, after movement to the dendrites of melanocytes, are released from there to the outside of the cell, and are involved in a membrane fusion between melanosomes and keratinocytes and in phagocytosis.

In relation to the synthesis process of melanin, it is ultimately synthesized from L-tyrosine through DOPA, DOPAquinone, DOPAchrome, and 5,6-dihydroxyindole (DHI). Traditionally, the inhibition of melanin synthesis was focused on factors that inhibit the activity of tyrosinase, which is the enzyme that catalyzes the rate-controlling step of the melanin synthesis pathway.

PCT International Publication WO 2020/153819 discloses a polypeptide having the activity of inhibiting the activity of tyrosinase, which is an important enzyme in the melanin synthesis pathway, and its use for skin whitening. Additionally, Korean Patent No. 10-1869783 discloses a peptide having the activity of inhibiting melanin production and tyrosinase activity and its use for skin whitening.

To date, the development of skin whitening agents has been focused on inhibiting the activity of tyrosinase, which is an important enzyme in melanin synthesis. However, in order to achieve a synergistic effect for skin whitening through various points of action, there is a need for the development of skin whitening agents with different mechanisms of action or points of action.

### [Prior Art Documents]

### [Patent Documents]

Patent Document 1. WO 2020/153819
Patent Document 2. Korean Patent No. 10-1869783

### DISCLOSURE OF THE INVENTION

### TECHNICAL PROBLEM

The present inventors have made research efforts to develop a peptide with improved activity and skin whitening activity. As a result, they have experimentally demonstrated that the novel peptide they have developed have an excellent effect of inhibiting the incorporation of melanosomes into keratinocytes and an excellent activity of degrading melanosomes, thereby completing the present invention.

Therefore, an object of the present invention is to provide a novel peptide with skin whitening activity.

Another object of the present invention is to provide a composition for skin whitening including the peptide having the above-described activity as an active ingredient.

Still another object of the present invention is to provide a pharmaceutical composition for the prevention or treatment of hyperpigmentation disease including the peptide having the above-described activity as an active ingredient.

Still another object of the present invention is to provide a cosmetic composition for skin whitening including the peptide having the above-described activity as an active ingredient.

### TECHNICAL SOLUTION

In order to achieve the above objects,
an aspect of the present invention provides a peptide including the amino acid sequence of SEQ ID NO: 1.

Another aspect of the present invention provides a composition for skin whitening including the peptide as an active ingredient.

Still another aspect of the present invention provides a pharmaceutical composition for preventing or treating hyperpigmentation disease, including the peptide as an active ingredient.

Still another aspect of the present invention provides a cosmetic composition for skin whitening including the peptide as an active ingredient.

The present invention will be described in detail below.

### 1. Peptides and activities thereof

According to an aspect of the present invention, a peptide including the amino acid sequence disclosed in SEQ ID NO: 1 is provided.

As used herein, the term "peptide" refers to a linear molecule formed by linking amino acid residues together through peptide bonds.

The peptide including the amino acid sequence of SEQ ID NO: 1 of the present invention may be used without modification, but within the range that does not affect the original activity of the peptide, such as skin whitening activity, variants which have different amino acid sequences or a fragment thereof by deletion, insertion, substitution of amino acid residues, or a combination thereof may be used.

The peptide of the present invention may be modified by phosphorylation, sulfation, acrylation, glycosylation, methylation, farnesylation, etc., within the range that its activities are not changed.

The peptide of the present invention includes a peptide including the amino acid sequence substantially identical to the peptide including the amino acid sequence of SEQ ID NO: 1, and a variant or active fragment thereof. The substantially identical amino acid sequence refers to an amino acid sequence having sequence identity of at least 75%, for example, at least 80%, at least 85%, at least 90%, at least 95%, and at least 97%, respectively, with the amino acid sequence of SEQ ID NO: 1. Additionally, the peptide may further include a targeting sequence, a tag, a labeled residue, and an amino acid sequence prepared for the specific purpose of increasing the half-life or stability of the peptide.

The peptide of the present invention may have N-terminal and/or C-terminal modifications induced to select a portion of the amino acid sequence and increase its activity. Through these N-terminal and/or C-terminal modifications, the stability of the peptide of the present invention can be significantly improved, for example, the half-life of the peptide can be increased when administered *in vivo.* The term "stability" is meant to include not only *in vivo* stability, which protects the peptide of the present invention from attack by protein-cleaving enzymes *in vivo,* but also storage stability (e.g., storage stability at room temperature).

The N-terminal modification may be the binding, to the N-terminus of the peptide, of a protective group selected from the group consisting of an acetyl group, a fluoreonylmethoxycarbonyl group, a formyl group, a palmitoyl group, a myristyl group, a stearyl group, and polyethylene glycol (PEG). The C-terminal modification may be the binding, to the C-terminus of the peptide, of a hydroxyl group (-OH), an amino group (-NH₂), an azide (-NHNH₂), etc., but is not limited thereto.

The peptide of the present invention may be prepared by various methods widely known in the technical field to which the present invention pertains. For example, the peptide of the present invention may be prepared using chemical synthesis methods known in the art, particularly solid-phase synthesis techniques (Merrifield, J. Amer. Chem. Soc. 85:2149-54(1963); Stewart, et al., Solid Phase Peptide Synthesis, 2nd. ed., Pierce Chem. Co.: Rockford, 111(1984)) or liquid phase synthesis technology (U.S. Pat. No. 5,516,891).

The peptide of the present invention has the activity of skin whitening.

In an embodiment, the peptide of the present invention has the activity of inhibiting the incorporation of melanosomes in keratinocytes or promoting the degradation of melanosomes.

Melanosomes produced in melanocytes move from melanocytes to keratinocytes. In particular, the movement of melanosomes to keratinocytes includes the process of releasing melanosomes from melanocytes and the process of incorporating melanosomes into keratinocytes. The incorporation process of melanosomes may be expressed differently as uptake of melanosomes by keratinocytes or phagocytosis of melanosomes by keratinocytes.

Protease activated receptor 2 (PAR2), Toll-like receptors 3 (TLR3), and keratinocyte growth factor receptor (KGFR) are involved in the absorption process of melanosomes released from melanocytes through phagocytosis by keratinocytes.

In one embodiment, the peptide of the present invention inhibits, in keratinocytes, the expression of one or more genes selected from the group consisting of the protease activated receptor 2 (PAR2) gene, Toll-like receptors 3 (TLR3) gene, and keratinocyte growth factor receptor (KGFR) gene.

The PAR2 is a receptor protein present in the cell membrane and is known to regulate inflammatory responses, obesity, metabolism, and cancer, and to act as a sensor for proteases during infection. The PAR2 protein is present in the membrane of keratinocytes and is involved in the absorption (incorporation) of melanosomes by keratinocytes. It is known that the inhibition of the activity of the PAR2 protein results in the inhibition of the absorption of melanosomes by keratinocytes (Exp Cell Res. 2000 Jan 10;254(1):25-32; Pigment Cell Res. 2001 Aug;14(4):236-42; Exp Dermatol. 2003;12 Suppl 2:5-12.).

The TLR3 protein is known to be a receptor protein that recognizes pathogens and plays an important role in activating innate immunity. It is known that stimulation of TRR 3 protein in keratinocytes induces improved absorption of melanosomes (J Dermatol Sci. 2019 Dec;96(3):168-177), and it is also known that in melanocytes, TRR3 protein promotes migration of melanosomes into keratinocytes (Int. J. Mol. Sci. 2020, 21, 9769).

The KGFR, which is a tyrosine kinase receptor, is known to be expressed in many types of epithelial cells, activated by four known ligands (*i.e*., FGF-1, FGF-3, FGF-7, and FGF-10), and is involved in proliferation and differentiation of epithelial cells, and wound recovery. In addition, KGFR is also known to promote the migration of melanosomes into keratinocytes (J Invest Dermatol. 2005 Dec; 125(6):1190-9., Journal of Investigative Dermatology Vol 128, Issue 3, March 2008, p. 558-567).

As described above, the peptide of the present invention can exhibit skin whitening activity and the effect of preventing or treating hyperpigmentation diseases through the activity of inhibiting of incorporation of melanosomes and the activity of degrading melanosomes.

### 2. Composition for skin whitening and preventing, treating, or improving hyperpigmentation diseases

In another aspect of the present invention, there is provided a composition for skin whitening including a peptide including the amino acid sequence of SEQ ID NO: 1 as an active ingredient.

The peptide including the amino acid sequence of SEQ ID NO: 1 of the present invention has skin whitening activity through the mechanism as described above.

In another aspect of the present invention, the composition of the present invention may be a pharmaceutical composition for preventing or treating hyperpigmentation diseases, which includes a peptide including the amino acid sequence of SEQ ID NO: 1 as an active ingredient.

In an embodiment, the pharmaceutical composition including the peptide as an active ingredient inhibits the incorporation of melanosomes into keratinocytes or promotes the degradation of melanosomes.

In an embodiment, the pharmaceutical composition including the peptide inhibits, in keratinocytes, the expression of one or more genes selected from the group consisting of the protease activated receptor 2 (PAR2) gene, Toll-like receptors 3 (TLR3) gene, and keratinocyte growth factor receptor (KGFR) gene.

As used herein, the term "hyperpigmentation disease" refers to a disease caused by an excessive increase in the amount of melanin in the skin. In this way, the skin color becomes darker due to an excessive increase in the amount of melanin in the skin. The causes of hyperpigmentation diseases may be, for example, exposure to sunlight, inflammation of the skin such as acne (post-inflammatory pigmentation), injury, hormonal imbalance, or medication, but are not limited thereto.

In an embodiment, the hyperpigmentation diseases may be melisma, freckles, age spots, solar lentigo, or post-inflammatory hyperpigmentation of the skin. The post-inflammatory hyperpigmentation may be hyperpigmentation that occurs after injury, ultraviolet rays, or cutaneous inflammatory disease such as acne.

The pharmaceutical composition of the present invention may include a therapeutically effective amount of the peptide including the amino acid of SEQ ID NO: 1 of the present invention.

The term "therapeutically effective amount" refers to an amount sufficient for the peptide, which is an active ingredient of the pharmaceutical composition of the present invention, to achieve its activity or efficacy, for example, a sufficient amount to achieve the efficacy of treating or preventing hyperpigmentation diseases.

As used herein, the term "prevention" refers to reducing the risk of developing a disease or disorder, and refers to all actions that inhibit or delay the onset of a disease by preventing the progression of the disease or one or more clinical symptoms thereof.

As used herein, the term "treatment" refers to alleviating a disease or disorder, and includes all actions that improve or beneficially change the symptoms of a disease by arresting or reducing the progression of the disease or one or more clinical symptoms thereof.

In the present invention, the prevention or treatment of hyperpigmentation disease may be removing the cause of hyperpigmentation in the skin or inhibiting the progression of hyperpigmentation, and specifically, it may be inhibiting the incorporation of melanosomes into keratinocytes or promoting the degradation of melanosomes.

The pharmaceutical composition of the present invention may include a pharmaceutically acceptable carrier.

The pharmaceutically acceptable carriers, which are commonly used in formulations, include lactose, dextrose, sucrose, sorbitol, mannitol, starch, gum arabic, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil, etc., but the carriers are not limited thereto.

In addition to the above ingredients, the pharmaceutical composition of the present invention may further include lubricants, wetting agents, sweeteners, flavoring agents, emulsifiers, suspending agents, preservatives, etc., but the ingredients are not limited thereto.

Suitable pharmaceutically acceptable carriers and formulations are described in detail in Remington: The Science and Practice of Pharmacy, (19th ed., 1995, Williams & Wilkins).

The pharmaceutical composition of the present invention may be administered by any route suitable for treating hyperpigmentation diseases, for example, orally or parenterally. In the case of parenteral administration, the composition may be administered by intravenous injection, subcutaneous injection, intramuscular injection, intraperitoneal injection, topical administration, transdermal administration, etc. Since the pharmaceutical composition of the present invention has the activity of preventing or treating hyperpigmentation diseases, it is desirable that the composition be applied topically, such as by applying it to the skin.

The dose of the pharmaceutical composition may be 0.0001 µg to 100 mg, 0.001 µg to 100 mg, 0.01 µg to 100 mg, 0.1 µg to 100 mg, or 1.0 µg to 1000 mg per day, but is not limited thereto. The pharmaceutical composition may be prescribed in various ways depending on factors, such as the formulation method, administration method, patient's age, weight, sex, pathological condition, food, administration time, administration route, excretion rate, and reaction sensitivity.

The pharmaceutical composition of the present invention may be prepared in unit dosage form by formulating it using a pharmaceutically acceptable carrier and/or excipient according to a method that can easily be implemented by those skilled in the art, or the pharmaceutical composition may be prepared by placing it in a multi-dose container. In particular, the formulation may be in the form of a solution, suspension, or emulsion in an oil or aqueous medium, or may be in the form of an extract, powder, granule, tablet, or capsule, and may additionally include a dispersant or stabilizer.

The pharmaceutical composition of the present invention may be an external skin preparation. The external skin preparation is a preparation that can be used by applying it to the outside of the skin. When the pharmaceutical composition of the present invention is used as an external skin preparation, it may be applied to the skin area where hyperpigmentation has occurred. The external skin preparation may be a cream, a gel, an ointment, a skin emulsifier, a skin suspension, a transdermal delivery patch, a drug-including bandage, a lotion, or a combination thereof. The skin external preparation may be appropriately mixed as needed with ingredients commonly used in skin external preparations (*e.g*., cosmetics and medicines), such as aqueous ingredients, oilbased ingredients, powder ingredients, alcohols, moisturizers, thickening agents, ultraviolet ray absorbers, whitening agents, preservatives, antioxidants, surfactants, fragrances, colorants, various skin nutrients, or combinations thereof. The skin external preparations may be appropriately mixed with metal sequestrants (*e.g*., disodium edetate, trisodium edetate, sodium citrate, sodium polyphosphate, sodium metaphosphate, gluconic acid, *etc*.), caffeine, tannin, a licorice extract, glabridin, various herbal medicines, tocopherol acetate, glycyrrhizinic acid, drugs (*e.g.,* tranexamic acid and derivatives or salts thereof, *etc*.), vitamin C, magnesium ascorbate phosphate, ascorbate glucoside, arbutin, kojic acid, and saccharides (*e.g*., glucose, fructose, trehalose, *etc*.), *etc.*

In another aspect of the present invention, the composition of the present invention provides a cosmetic composition for skin whitening including a peptide, which includes the amino acid sequence of SEQ ID NO: 1, as an active ingredient.

In an embodiment, the cosmetic composition including the peptide of the present invention as an active ingredient inhibits the incorporation of melanosomes into keratinocytes or promotes the degradation of melanosomes.

In an embodiment, the cosmetic composition including the peptide of the present invention as an active ingredient inhibits the expression of one or more genes selected from the group consisting of the protease activated receptor 2 (PAR2) gene, Toll-like receptors 3 (TLR3) gene, and keratinocyte growth factor receptor (KGFR) gene.

The cosmetic composition may be prepared in any formulation commonly prepared in the technical field to which the present invention pertains, and may be an external skin preparation. For example, the cosmetic composition may be formulated as a solution, a suspension, an emulsion, a paste, a gel, a cream, a lotion, powder, a soap, a surfactant-including cleansing, an oil, a powder foundation, an emulsion foundation, a wax foundation, a spray, *etc*., but is not limited thereto.

The cosmetic composition may be prepared in various forms, such as a solution (*e.g.,* a softening lotion, a nourishing lotion, a nourishing cream, a massage cream, an essence, an eye cream, a cleansing cream, a cleansing foam, cleansing water, a pack, a spray, powder, a hair tonic, a hair cream, a hair lotion, a hair shampoo, a hair rinse, a hair conditioner, a hair spray, a hair aerosol, a pomade, a gel, *etc*.), a sol-gel, an emulsion, an oil, a wax, an aerosol, etc., but is not limited thereto.

The cosmetic composition of the present invention may include other additives, such as excipients and carriers, and it is possible to apply and mix the usual ingredients mixed in general skin cosmetics as necessary.

When the formulation of the cosmetic composition is a paste, a cream, or a gel, it is possible to use, as a carrier ingredient, an animal oil, a vegetable oil, a wax, paraffin, starch, tragacanth, a cellulose derivative, polyethylene glycol, silicone, bentonite, silica, talc, zinc oxide, etc. may be used.

When the formulation of the cosmetic composition is powder or a spray, it is possible to use, as a carrier ingredient, lactose, talc, silica, aluminum hydroxide, calcium silicate, or polyamide powder, and particularly, when the cosmetic composition is a spray, it is possible to further include a propellant, such as chlorofluorohydrocarbon, propane/butane, or dimethyl ether, but is not limited thereto.

When the cosmetic composition is in the form of a solution or emulsion, it is possible to use, as a carrier ingredient, a solvent, a solubilizing agent, or an emulsifying agent, for example, water, ethanol, isopropanol, ethyl carbonate, ethyl acetate, benzyl alcohol, benzyl benzoate, propylene glycol, 1,3-butyl glycol oil, glycerol aliphatic ester, polyethylene glycol, sorbitan fatty acid ester, etc.

When the formulation of the cosmetic composition is a suspension, it is possible to use, as a carrier ingredient, a liquid-phase diluent (*e.g*., water, ethanol, and propylene glycol), a suspending agent (*e.g*., ethoxylated isostearyl alcohol, polyoxyethyl sorbitol ester, polyoxyethylene sorbitan ester, *etc*.), microcrystalline cellulose, aluminum metahydroxide, bentonite, agar, tragacanth, *etc*.

When the formulation of the cosmetic composition is a surfactant-including cleansing agent, it is possible to use, as a carrier ingredient, aliphatic alcohol sulfate, aliphatic alcohol ether sulfate, sulfosuccinic acid monoester, isethionate, an imidazolinium derivative, methyl taurate, sarcosinate, fatty acid amide ether sulfate, alkylamidobetaine, aliphatic alcohol, fatty acid glyceride, fatty acid diethanolamide, vegetable oil, a lanolin derivative, ethoxylated glycerol fatty acid ester, *etc*.

When the formulation of the cosmetic composition is a hair shampoo, the peptide of the present invention may be mixed with base ingredients for forming a shampoo, such as a thickener, a surfactant, a viscosity modifier, a moisturizer, a pH adjuster, a preservative, and an essential oil. CDE may be used as the thickener; LES, an anionic surfactant, and coco betaine, an amphoteric surfactant, may be used as the surfactant; polyquarter may be used as the viscosity modifier; glycerin may be used as the moisturizer; and citric acid and sodium hydroxide may be used as the pH adjuster. A grapefruit extract, *etc*. may be used as the preservative; additionally, essential oils of cedarwood, peppermint, and rosemary, and silk amino acids, pentaol, or vitamin E may be added.

The ingredients to be included in the cosmetic composition as active ingredients may include, in addition to the peptide and carrier ingredients of the present invention, auxiliary ingredients commonly used in cosmetic compositions, such as antioxidants, stabilizers, solubilizers, vitamins, pigments, and fragrances, but are not limited thereto.

The peptide of the present invention may be included in the above-described composition, pharmaceutical composition, or cosmetic composition at a concentration of 0.01 µM to 1000 µM, and specifically, the peptide of the present invention may be included at a concentration of 0.01 µM to 1000 µM; 0.05 µM to 800 µM, 0.05 µM to 700 µM, 0.05 µM to 600 µM, 0.05 µM to 500 µM, 0.05 µM to 300 µM, or 0.05 µM to 200 µM; 0.1 µM to 800 µM, 0.1 µM to 700 µM, 0.1 µM to 600 µM, 0.1 µM to 500 µM, 0.1 µM to 300 µM, or 0.1 µM to 200 µM; 1 µM to 800 µM, 1 µM to 700 µM, 1 µM to 600 µM, 1 µM to 500 µM, 1 µM to 300 µM, or 1 µM to 200 µM; or 5 µM to 800 µM, 5 µM to 700 µM, 5 µM to 600 µM, 5 µM to 500 µM, 5 µM to 300 µM, or 5 µM to 200 µM, but is not limited thereto.

### 3. Use of peptide of present invention

In another aspect of the present invention, a peptide including the amino acid sequence of SEQ ID NO: 1 is provided for use in skin whitening or for preventing, treating, or improving hyperpigmentation diseases.

In another aspect of the present invention, there is provided a skin whitening method, which includes administering a peptide including the amino acid sequence of SEQ ID NO: 1 or a composition including the peptide to a subject in need of skin whitening.

In still another aspect of the present invention, there is provided a method for preventing or treating hyperpigmentation diseases, which includes administering a peptide including the amino acid sequence of SEQ ID NO: 1 or a composition including the peptide to a subject in need of prevention or treatment of hyperpigmentation diseases.

In still another aspect of the present invention, there is provided a use of the peptide including the amino acid sequence of SEQ ID NO: 1 for the preparation of a medicament for the prevention or treatment of hyperpigmentation diseases.

In still another aspect of the present invention, there is provided a use of a peptide including the amino acid sequence of SEQ ID NO: 1 for the preparation of cosmetics for skin whitening.

### ADVANTAGEOUS EFFECTS

The peptide of the present invention exhibits the activity of skin whitening by inhibiting the incorporation of melanosome in keratinocytes and promoting the degradation of melanosomes. Accordingly, the peptide of the present invention can be used as an active ingredient in drugs for the treatment or prevention of hyperpigmentation diseases caused by the deposition of excessive melanosome, or as an effective ingredient in cosmetics for skin whitening.

However, the effects of the present invention are not limited to the effects mentioned above, and other effects not mentioned will be clearly understood by those skilled in the art from the following description.

### BRIEF DESCRIPTION OF THE DRAWINGS

FIG. 1 shows the results of a melanin content analysis illustrating that the peptide of the present invention inhibits the incorporation of melanosomes in HaCaT cells.
FIG. 2a shows the results of a PCR analysis illustrating that the peptide of the present invention reduces the mRNA level of PAR2, which is a receptor related to melanosome incorporation, in a concentration-dependent manner in HaCaT cells.
FIG. 2b shows a graph illustrating the measured values of a band density of the electrophoresis of FIG. 2a.
FIG. 3a shows the results of a PCR analysis illustrating that the peptide of the present invention reduces the mRNA level of TLR3, which is a receptor related to incorporation of melanosomes in HaCaT cells.
FIG. 3b shows a graph illustrating the measured values of a band density of the electrophoresis of FIG. 3a.
FIG. 4a shows the results of a PCR analysis illustrating that the peptide of the present invention reduces the mRNA level of KGFR, which is a receptor related to incorporation of melanosomes in HaCaT cells.
FIG. 4b shows a graph illustrating the measured band density of the electrophoresis of FIG. 4a.

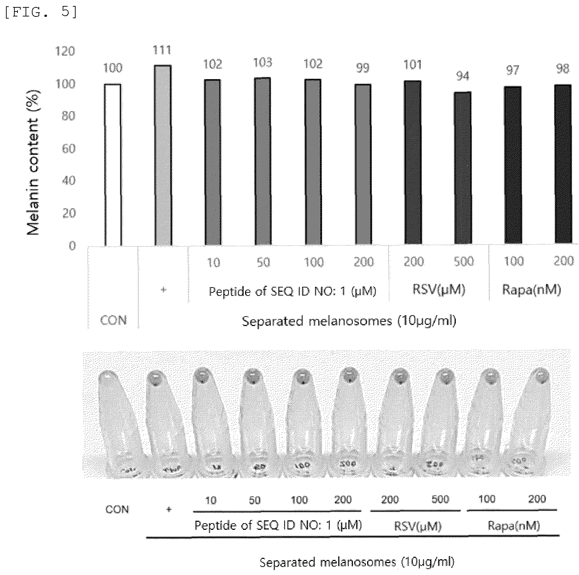

FIG. 5 shows the results of a melanosome content analysis illustrating that the peptide of the present invention induces the degradation of melanosomes in HaCaT cells.

### MODE FOR CARRYING OUT THE INVENTION

Hereinafter, the present invention will be described in detail. However, the following examples specifically illustrate the present invention, and the content of the present invention is not limited by the following examples.

### Preparation Example 1: Preparation of peptide

A peptide having the amino acid sequence of SEQ ID NO: 1 shown in Table 1 below was synthesized using an automatic peptide synthesizer (Milligen 9050, Millipore, USA), and these synthesized peptides were then purified using C18 reversed-phase high performance liquid chromatography (HPLC) (Waters Associates, USA). The column used was ACQUITY UPLC BEH300 C18 (2.1 mm × 100 mm, 1.7 µm, Waters Co, USA).

**[Table 1]**

| SEQ ID NO: | Amino acid sequence of peptide |
|---|---|
| 1 | RTLE (Arg-Thr-Leu-Glu) |

The efficacy of the peptide of SEQ ID NO: 1 prepared was evaluated through the following experiments.

### Experimental Example 1: Analysis of incorporation by phagocytosis of melanosomes

It was confirmed whether the peptide of SEQ ID NO: 1 prepared in Preparation Example 1 inhibits the incorporation of melanosomes by phagocytosis in keratinocytes.

Human keratinocyte cell line (HaCaT cells) was seeded in a 6-well cell culture plate at a density of 3 × 10⁵ cells/well and cultured in Dulbecco's Modified Eagle's Medium (DMEM) including 10% FBS for 24 hours. Then, the cells were washed once with serum free DMEM. A solution of the peptide of SEQ ID NO: 1 of Preparation Example 1 was added to 3 mL of serum-free DMEM to prepare the peptide solution at concentrations of 50 µM, 100 µM, and 200 µM, and the solution was each added to cultured cells. In particular, the negative control group (CON, (+)) was an untreated group, and the positive control group was treated with 500 nM of 6-amino-1-[4-(3-methyl-1-oxobutyl)-1-piperazinyl]-1-hexanone hydrochloride (ENMD-1068, Sigma-Aldrich). Then, after culturing them in a CO₂ incubator at 37°C for 24 hours, melanosomes (10 µg/mL) isolated from human epidermal melanocyte-darkly pigmented donors (HEM-DP) were additionally added to the cultured cells excluding the negative control (CON) group. Thereafter, the cells were cultured in a CO₂ incubator at 37°C for 48 hours, washed three times with PBS, treated with 1X TE for 10 minutes, and the HaCaT cells were collected. The HaCaT cells were recovered using a centrifuge, dissolved in 1 M NaOH, and dispensed into a 96-well plate. The melanin content was measured by the absorbance at 490 nm using an ELISA reader.

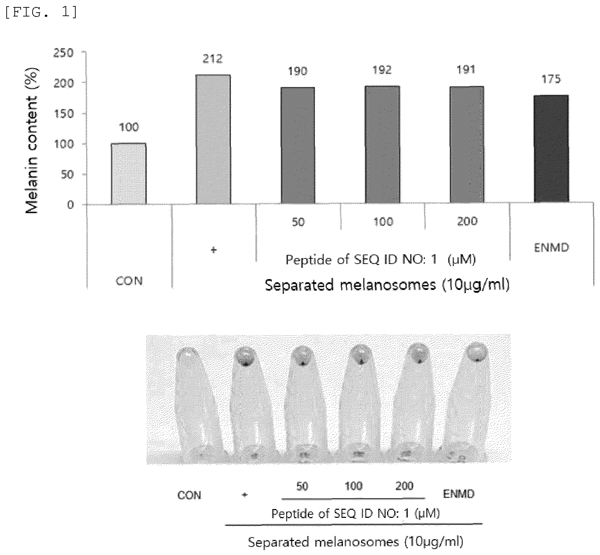

As a result of the experiment, as can be seen from the results of the melanin content analysis shown in FIG. 1, it was confirmed that the incorporation of melanosomes into HaCaT cells was inhibited by the peptide of SEQ ID NO: 1.

### Experimental Example 2: Analysis of expression of PAR2 gene related to melanosome phagocytosis

An experiment was performed regarding the effect of the peptide of SEQ ID NO: 1 prepared in Preparation Example 1 on the expression of protease activated receptor 2 (PAR2), which is the melanosome phagocytosis-related gene, in keratinocytes.

HaCaT cells were seeded in a 6-well plate at a density of 3 × 10⁵ cells/well and cultured in DMEM medium including 10% FBS for 24 hours. Then, the cells were washed once with serum-free DMEM medium, and then the peptide of SEQ ID NO. 1 of Preparation Example 1 was added to 3 mL of serum-free DMEM medium to prepare the peptide solution at concentrations of 10 uM, 50 µM, and 100 µM, and the solution was each dispensed into the cells. In particular, the negative control group (CON, (+)) was an untreated group, and the positive control group was treated with 0.02X and 0.05X protease inhibitor (PI) (cOmplete^{™} Protease Inhibitor Cocktail, Roche, CH). Then, after culturing in a CO₂ incubator at 37°C for 24 hours, 4 units of trypsin were added to the cultured cells excluding the negative control (CON) group. Thereafter, the cells were cultured in a CO₂ incubator at 37°C for 16 hours, washed two times with PBS, and RNA was isolated using easy blue (iNtRON, Cat. No.: 17061, Korea). After quantifying the amount of isolated RNA, 2000 ng of RNA was dispensed per tube and cDNA was synthesized using a cDNA synthesis kit (Enzynomics, Cat. No.: RT200, Korea). PCR was performed using primers targeting the PAR2 gene shown in Table 2 and a PCR kit (Enzynomics, Cat. No.: P581T, Korea). Next, the PCR product was subjected to electrophoresis on a 1.2% agarose gel, and the bands were detected and analyzed using the Bio-Rad gel image system.

**[Table 2]**

| Primer name | Sequence (5'→3') | SEQ ID NO: |
|---|---|---|
| PAR2 Forward | (5') TGC TAG CAG CCT CTC TCT CC (3') | 2 |
| PAR2 Reverse | (5') CTT CAA GGG GAA CCA GAT GA (3') | 3 |
| GAPDH Forward | (5') GGA GCC AAA AGG GTC ATC AT (3') | 4 |
| GAPDH Reverse | (5') GTG ATG GCA TGG ACT GTG GT (3') | 5 |

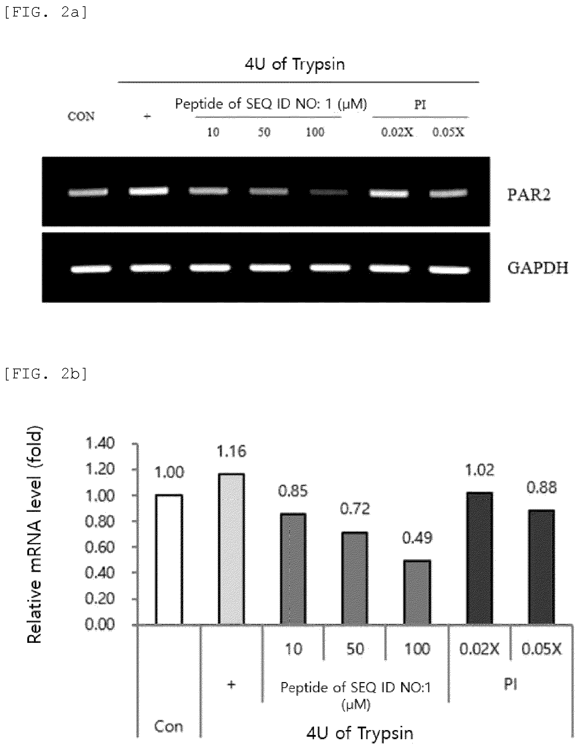

As a result of the experiment, as shown in FIGS. 2a and 2b, it was confirmed that the peptide of SEQ ID NO: 1 reduced the mRNA level of the PAR2 gene, which is a receptor related to melanosome phagocytosis (incorporation), in a concentration-dependent manner in HaCaT cells. These results confirmed that the peptide of SEQ ID NO: 1 inhibits the incorporation of melanosomes.

### Experimental Example 3: Expression analysis of TLR3 gene related to melanosome phagocytosis

An experiment was performed regarding the effect of the peptide of SEQ ID NO: 1 prepared in Preparation Example 1 on the expression of Toll-like receptors 3 (TLR3), which is a gene related to melanosome phagocytosis.

HaCaT cells were seeded in a 6-well plate at a density of 3 × 10⁵ cells/well and cultured in DMEM medium including 10% FBS for 24 hours. Then, the cells were washed once with serum-free DMEM medium, and then 2 mL of serum-free DMEM medium was added to the cells. The cells were then cultured in a CO₂ incubator at 37°C for 24 hours. The peptide of SEQ ID NO: 1 of Preparation Example 1 was added to 3 mL of DMEM medium including 2% serum to prepare the peptide solution at concentrations of 10 µM, 50 µM, 100 µM, and 200 µM, and this solution was each added to the cells. After 30 minutes, 30 µg/mL of poly(I:C) was additionally added thereto. Subsequently, the cells were cultured in a CO₂ incubator at 37°C for 24 hours, washed twice with PBS, and RNA was isolated using easy blue (iNtRON, Cat. No.: 17061, Korea). After quantifying the amount of isolated RNA, 2000 ng of RNA was dispensed per tube and cDNA was synthesized using a cDNA synthesis kit (Enzynomics, Cat. No.: RT200, Korea). PCR was performed using primers targeting the TLR3 gene in Table 3 and a PCR kit (Enzynomics, Cat. No.: P581T, Korea). Then, the PCR product was subjected to electrophoresis on a 1.2% agarose gel, and the bands were detected and analyzed using the Bio-Rad gel image system.

**[Table 3]**

| Primer name | Sequence (5'->3') | SEQ ID NO: |
|---|---|---|
| TLR3 Forward | (5') GCC GTC TAT TTG CCA CAC AC (3') | 6 |
| TLR3 Reverse | (5') GCA GTC AGC AAC TTC ATG GC (3') | 7 |
| GAPDH Forward | (5') GGA GCC AAA AGG GTC ATC AT (3') | 4 |
| GAPDH Reverse | (5') GTG ATG GCA TGG ACT GTG GT (3') | 5 |

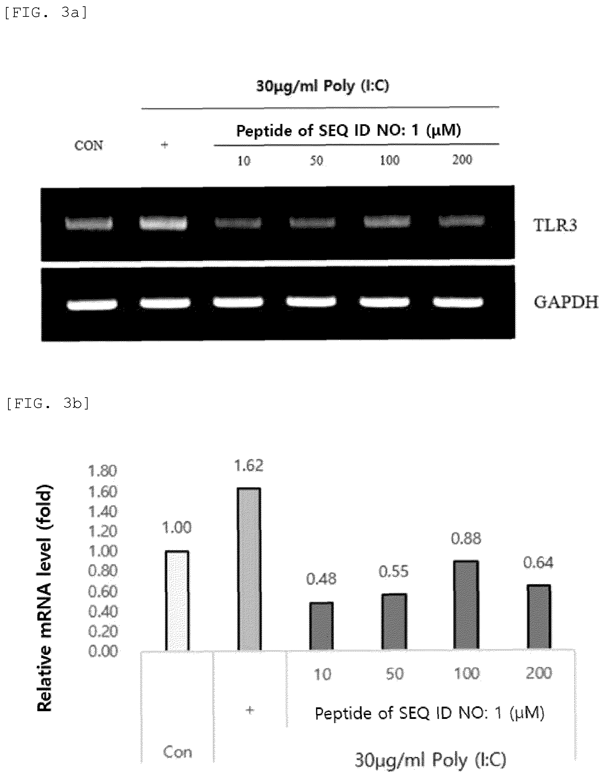

As a result of the experiment, as shown in FIGS. 3a and 3b, it was confirmed that the peptide of SEQ ID NO: 1 reduced the mRNA level of the gene of TLR3, which is a receptor related to melanosome phagocytosis (incorporation), in HaCaT cells compared to the control group. These results confirmed that the peptide of SEQ ID NO: 1 inhibits the incorporation of melanosomes.

### Experimental Example 4: Expression analysis of KGFR gene related to melanosome phagocytosis

An experiment was performed regarding the effect of the peptide of SEQ ID NO: 1 prepared in Preparation Example 1 on the expression of keratinocyte growth factor receptor (KGFR), which is a melanosome phagocytosis-related gene.

HaCaT cells were seeded in a 6-well plate at a density of 3 × 10⁵ cells/well and cultured in DMEM medium including 10% FBS for 24 hours. Then, the cells were washed once with serum-free DMEM medium, and then 2 mL of serum-free DMEM medium was added to the cells. The cells were then cultured in a CO₂ incubator at 37°C for 24 hours. The peptide of SEQ ID NO: 1 of Preparation Example 1 was added to 3 mL of DMEM medium including 2% serum to prepare the peptide solution at concentrations of 10 µM, 50 µM, 100 µM, and 200 µM, and this solution was each added to the cells and the cells were cultured in a CO₂ incubator at 37°C for 6 hours. Subsequently, after washing the cells twice with PBS, RNA was isolated using easy blue (iNtRON, Cat. No.: 17061, Korea). After quantifying the amount of isolated RNA, RNA was dispensed in an amount of 2000 ng per tube and cDNA was synthesized using a cDNA synthesis kit (Enzynomics, Cat. No.: RT200, Korea). PCR was performed using primers targeting the KGFR gene in Table 4 and a PCR kit (Enzynomics, Cat. No.: P581T, Korea). Then, the PCR product was subjected to electrophoresis on a 1.2% agarose gel, and the bands were detected and analyzed using the Bio-Rad gel image system.

**[Table 4]**

| Primer name | Sequence (5'->3') | SEQ ID NO: |
|---|---|---|
| KGFR Forward | (5') TGA CCA AAC GTA TCC CCC TG (3') | 8 |
| KGFR Reverse | (5') GGT GTC TGC CGT TGA AGA GA (3') | 9 |
| GAPDH Forward | (5') GGA GCC AAA AGG GTC ATC AT (3') | 4 |
| GAPDH Reverse | (5') GTG ATG GCA TGG ACT GTG GT (3') | 5 |

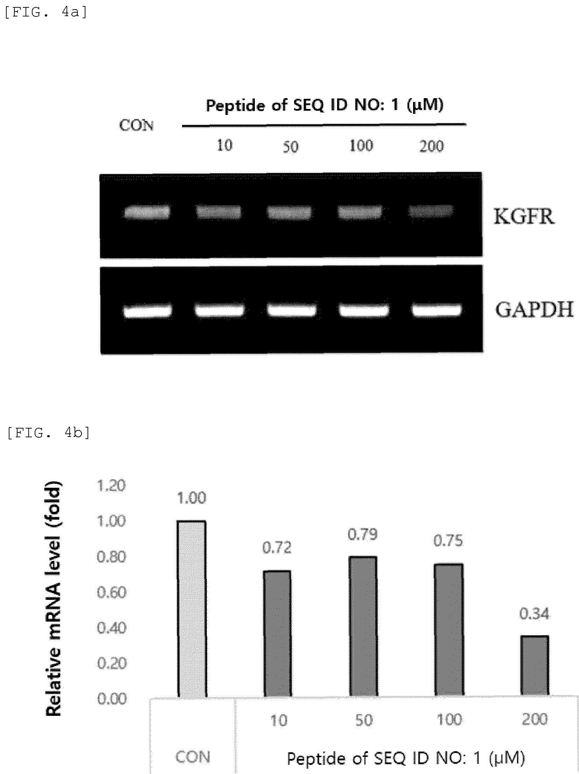

As a result of the experiment, as shown in FIGS. 4a and 4b, it was confirmed that the peptide of SEQ ID NO: 1 reduced the mRNA level of the gene of KGFR, which is a receptor related to melanosome phagocytosis, in HaCaT cells compared to the control group. These results confirmed that the peptide of SEQ ID NO: 1 inhibits the incorporation of melanosomes.

### Experimental Example 5: Melanosome degradation analysis

An analysis was performed regarding whether the peptide of SEQ ID NO: 1 prepared in Preparation Example 1 degrades melanosomes.

HaCaT cells were seeded in a 6-well plate at a density of 3 × 10⁵ cells/well and cultured in DMEM medium including 10% FBS for 24 hours. Then, melanosomes isolated from human epidermal melanocyte-darkly pigmented donors (HEM-DP) were added to 2 mL of DMEM medium including 2% serum to prepare a melanosome solution at a concentration of 10 µg/mL, which was dispensed into cells, and the cells were incubated in a CO₂ incubator at 37° for 48 hours.

Subsequently, melanosomes isolated from HEM-DP were added to 3 mL of DMEM medium including 2% serum to prepare a melanosome solution with a concentration of 10 µg/mL, and the peptide of SEQ ID NO: 1 of Preparation Example 1 was added to 3 mL of DMEM medium including 2% serum to prepare the peptide solution at concentrations of 10 µM, 50 µM, 100 µM, and 200 µM, and the melanosome solution and the peptide solution were additionally dispensed into the cells. In particular, the negative control group (CON) was an untreated group, in which the negative control group (+) was treated only with a melanosome solution, whereas the positive control group included the groups (Rapa) treated with a melanosome solution and rapamycin at 100 nM and 200 nM, and the groups (RSV) treated with a melanosome solution and resveratrol at 200 µM and 500 µM. Then, the cells were cultured in a CO₂ incubator at 37° for 72 hours, washed three times with PBS, and then treated with 1X TE for 10 minutes, and the HaCaT cells were collected. The HaCaT cells were recovered using a centrifuge, dissolved in 1 M NaOH, and dispensed into a 96-well plate. The amount of melanin was measured by the absorbance at 490 nm using an ELISA reader.

As a result of the experiment, as can be seen from the change in melanosome content in FIG. 3, it was confirmed that treatment with the peptide of SEQ ID NO: 1 induces the degradation of melanosomes in HaCaT cells.

### Preparation Example 2: Preparation of pharmaceutical composition

### 2-1. Preparation of ointments

the peptide of the present invention 5 g
cetyl palmitate 20 g
cetanol 40 g
stearyl alcohol 40 g
myristane isopropyl 80 g
polysorbate 60 g
propyl paraoxybenzoate 1 g
methyl paraoxybenzoate 1 g
phosphoric acid and purified water an adequate amount

The ointment was prepared by incorporating the above ingredients at the specified contents according to a conventional ointment preparation method.

### 2-2. Preparation of powder

the peptide of the present invention 2 g
lactose 1 g

The powder was prepared by mixing the above ingredients followed by filling the mixture into an airtight pouch.

### 2-3. Preparation of tablets

the peptide of the present invention 100 mg
corn starch 100 mg
lactose 100 mg
magnesium stearate 2 mg

Tablets were prepared by mixing the above ingredients followed by tableting the mixture according to a conventional tablet preparation method.

### 2-4. Preparation of capsules

the peptide of the present invention 100 mg
corn starch 100 mg
lactose 100 mg
magnesium stearate 2 mg

Capsules were prepared by mixing the above ingredients followed by filling the mixture into gelatin capsules according to a conventional capsule preparation method.

### 2-5. Preparation of pills

the peptide of the present invention 1 g
lactose 1.5 g
glycerin 1 g
xylitol 0.5 g

After mixing the above ingredients, pills were prepared in an amount of 4 g per pill according to a conventional method.

### Preparation Example 3: Preparation of cosmetic composition

### 3-1. Preparation of cream

the peptide of the present invention 4.6 parts by weight
cetostearyl alcohol 2.8 parts by weight
beeswax 2.6 parts by weight
stearic acid 1.4 parts by weight
lipophilic glycerin monostearate 2 parts by weight
PEG-100 stearate 1 part by weight
sorbital sesquioleate 1.4 parts by weight
jojoba oil 4 parts by weight
squalane 3.8 parts by weight
polysorbate 60 1.1 parts by weight
macadamia oil 2 parts by weight
tocopherol acetate 0.2 parts by weight
methylpolysiloxane 0.4 parts by weight
ethylparaben 0.1 parts by weight
propylparaben 0.1 parts by weight
Euxyl K-400 0.1 parts by weight
1,3-butylene glycol 7 parts by weight
methylparaben 0.05 parts by weight
glycerin 6 parts by weight
d-panthenol 0.2 parts by weight
triethanolamine 0.2 parts by weight
pt 41891 0.2 parts by weight
p-H₂O 46.05 parts by weight

### 3-2. Preparation of lotions

the peptide of the present invention 3.5 parts by weight
cetostearyl alcohol 1.6 parts by weight
stearic acid 1.4 parts by weight
lipophilic glycerin monostearate 1.8 parts by weight
PEG-100 stearate 2.6 parts by weight
sorbital sesquioleate 0.6 parts by weight
squalene 4.8 parts by weight
macadamia oil 2 parts by weight
jojoba oil 2 parts by weight
tocopherol acetate 0.4 parts by weight
methylpolysiloxane 0.2 parts by weight
ethylparaben 0.1 parts by weight
propylparaben 0.1 parts by weight
1,3-butylene glycol 4 parts by weight
methylparaben 0.1 parts by weight
xanthan gum 0.1 parts by weight
glycerin 4 parts by weight
d-panthenol 0.15 parts by weight
allantoin 0.1 parts by weight
calcium carbonate (2% aq. Sol) 4 parts by weight
triethanolamine 0.15 parts by weight
ethanol 3 parts by weight
pt 41891 0.1 parts by weight
p-H₂0 48.3 parts by weight

### 3-3. Preparation of softening lotions

the peptide of the present invention 0.2 wt%
ethanol 10.0 wt%
polyoxyethylene sorbitan polylaurate 1.0 wt%
methyl paraoxybenzoate 0.2 wt%
glycerin 5.0 wt%
1,3-butyl glycol 6.0 wt%
fragrance an adequate amount
pigment an adequate amount
purified water an adequate amount
Total 100

### 3-4. Preparation of nourishing lotions

the peptide of the present invention 0.1 wt%
vaseline 2.0 wt%
sorbitan sesquioleate 0.8 wt%
polyoxyethylene oleyl ethyl 1.2 wt%
methyl paraoxybenzoate an adequate amount
propylene glycol 5.0 wt%
ethanol 3.2 wt%
carboxyvinyl polymer 18.0 wt%
pigment an adequate amount
fragrance an adequate amount
purified water an adequate amount
Total 100

### 3-5. Preparation of essences

the peptide of the present invention 5.0 wt%
propylene glycol 10.0 wt%
glycerin 10.0 wt%
an aqueous solution of sodium hyaluronate (1%) 5.0 wt%
ethanol 3.2 wt%
polyoxyethylene hydrogenated castor oil 1.0 wt%
methyl paraoxybenzoate 0.1 wt%
fragrance an adequate amount
purified water an adequate amount
Total 100

### 3-6. Preparation of packs

the peptide of the present invention 0.5 wt%
glycerin 5.0 wt%
propylene glycol 4.0 wt%
polyvinyl alcohol 15.0 wt%
ethanol 8.0 wt%
polyoxyethylene oleyl ethyl 1.0 wt%
methyl paraoxybenzoate 0.2 wt%
fragrance an adequate amount
pigment an adequate amount
purified water an adequate amount
Total 100

The above composition ratio is a mixture of suitable ingredients in preferred embodiments, but the ingredients or mixing ratios thereof may be arbitrarily modified according to regional or ethnic preferences such as demand segments, countries of demand, and intended uses.

In the above, representative embodiments of the present application have been described by way of exemplary embodiments, but the scope of the present application is not limited to the specific embodiments described above, and those skilled in the art will be able to make appropriate modifications within the scope described in the claims of this application.

## Claims

1. A peptide comprising an amino acid sequence of SEQ ID NO: 1.

2. A composition for skin whitening comprising the peptide of claim 1 as an active ingredient.

3. A pharmaceutical composition for preventing or treating hyperpigmentation diseases comprising the peptide of claim 1 as an active ingredient.

4. The pharmaceutical composition of claim 3, wherein in keratinocytes, the peptide:
(i) inhibits the incorporation of melanosomes; or
(ii) promotes the degradation of melanosomes.

5. The pharmaceutical composition of claim 4, wherein in keratinocytes, the peptide inhibits the expression of one or more genes selected from the group consisting of the protease activated receptor 2 (PAR2) gene, Toll-like receptors 3 (TLR3) gene, and keratinocyte growth factor receptor (KGFR) gene.

6. The pharmaceutical composition of claim 3, wherein the hyperpigmentation diseases are diseases that occur when the amount of melanin in the skin increases excessively.

7. The pharmaceutical composition of claim 3, wherein the hyperpigmentation diseases are melisma, freckles, age spots, solar lentigo, or post-inflammatory hyperpigmentation of the skin.

8. The pharmaceutical composition of claim 3, wherein the pharmaceutical composition is a skin preparation for external use.

9. A cosmetic composition for skin whitening comprising the peptide of claim 1 as an active ingredient.

10. The cosmetic composition of claim 9, wherein in keratinocytes, the peptide:
(i) inhibits the incorporation of melanosomes; or
(ii) promotes the degradation of melanosomes.

11. The cosmetic composition of claim 10, wherein in keratinocytes, the peptide inhibits the expression of one or more genes selected from the group consisting of the protease activated receptor 2 (PAR2) gene, Toll-like receptors 3 (TLR3) gene, and keratinocyte growth factor receptor (KGFR) gene.

12. The cosmetic composition of claim 9, wherein the cosmetic composition is a skin preparation for external use.

13. The cosmetic composition of claim 12, wherein the cosmetic composition is at least one formulation selected from the group consisting of a solution, a suspension, an emulsion, a paste, a gel, a cream, a lotion, a powder, a soap, a surfactant-including cleansing, an oil, a powder foundation, an emulsion foundation, a wax foundation, and a spray.
